# EUROPEAN PATENT APPLICATION

(11) **EP 4 272 803 A1**
(43) Date of publication of application: **08.11.2023**
(21) Application number: 21913468.1
(22) Date of filing: 04.11.2021
(51) Int. Cl.: A61M 25/10

(54) **EXPANSION SACCULUS AND SACCULUS EXPANSION CATHETER**

(30) Priority: 30.12.2020 CN 202011611933
(71) Applicant: Shanghai MicroPort CardioFlow Medtech Co., Ltd., Shanghai 201203 (CN)
(72) Inventor: YUE, Bin, Shanghai 201203 (CN); ZHAO, Ruoheng, Shanghai 201203 (CN); YAO, Yingzhong, Shanghai 201203 (CN); ZHANG, Jingyi, Shanghai 201203 (CN); CHANG, Zhaohua, Shanghai 201203 (CN)
(74) Representative: Lavoix
(86) International application number: PCT/CN2021/128827
(87) International publication number: WO 2022/142724

(57) **Abstract**

An expansion balloon (1) and a balloon expansion catheter, wherein the expansion balloon (1) includes an outer balloon (10) and an inner balloon (20). The outer balloon (10) is sleeved over the inner balloon (20), and the inner balloon (20) can switch between a collapsed configuration, a pre-shaped configuration and a first expanded configuration. The outer balloon (10) has a second expanded configuration. When an inflation fluid is filled into the inner balloon (20) in a configuration between the collapsed configuration and the pre-shaped configuration, the inner balloon (20) will be expanded and transition from the collapsed configuration toward the pre-shaped configuration, with the outer balloon (10) being expanded with the inner balloon (20). When the inflation fluid is additionally filled into the inner balloon (20) in the pre-shaped configuration, the inner balloon (20) will be further expanded, resulting in a further expansion of the outer balloon (10), until the outer balloon (10) comprises the second expanded configuration, where the inner balloon (20) is in the first expanded configuration. With this design, when in the pre-shaped configuration, the inner balloon (20) can satisfy anchoring of various diseased sites, partial expansion of various diseased sites and special balloon shape requirements. The outer balloon (10) defines a final expanded configuration of the expansion balloon (1), avoiding complications caused by excessive expansion.

## Description

### TECHNICAL FIELD

The present invention relates to the field of medical devices and, in particular, to expansion balloons and a balloon expansion catheter.

### BACKGROUND

Aortic stenosis (AS) is one of the most common valvular heart conditions and seriously affect human's health. For patients with severe aortic stenosis, surgical aortic valve replacement used to be the only way of extending their lives. However, elderly patients are often contraindicated for surgery due to advanced age, weak constitution, a severe condition or complications. More and more clinical results have confirmed that interventional treatment is effective for high-risk patients or patients for whom traditional thoracotomy would be too risky. Currently available interventional therapies include balloon aortic valvuloplasty (BAV) and transcatheter aortic valve implantation (TAVI). Both BAV and TAVI employ balloon expansion catheters. In a BAV procedure, a balloon is used to directly expand a diseased native aortic valve. In a TAVI procedure, a balloon is used for primary expanding the calcified native valvular annulus and creating desired conditions for access of an artificial valve to be implanted. Moreover, after the artificial aortic valve is implanted, the balloon is further used for secondary expansion so as to sufficiently expand the artificial valve so that it fits snugly against the wall of the blood vessel, resulting in better therapeutic outcomes.

A balloon expansion catheter for use with an aortic valve is composed of an expandable valve balloon, a double-lumen catheter and two connecting members. The valve balloon is desired to have low compliance, dimensional stability, fast expansion and withdrawal, puncture resistance, burst resistance, resistance to unwanted sudden movement, synchronous expansion and other capabilities. An inner lumen of the double-lumen catheter has a size compatible with a size of a corresponding guidewire, and an outer lumen is required to be unobstructed to minimize the times required for inflation and withdrawal.

Most existing valve balloons are designed as a straight or figure-eight shaped cylinder, and the inner tube comprises a round hollow tube. The straight cylindrical design can facilitate uniform expansion of the balloon at a lesion side and avoid tearing of valve leaflets during excessive expansion. However, during expansion, a straight cylindrical balloon is prone to unwanted sudden movement when squeezed by severely calcified leaflets. When this happens, the balloon will shift away from the diseased site and may fail to expand the diseased leaflets. The figure-eight shaped design has a waist section capable of anchoring the balloon at a diseased site, reducing to a certain extent the probability of unwanted sudden movement. Existing figure-eight shaped balloons can be divided into the two types as follows:
Type I: In order to prevent unwanted sudden movement, a balloon of this type has a waist section, which persists from a collapsed configuration to a completely expanded configuration of the balloon. In the completely expanded configuration, there is a noticeable diameter difference between the waist section and either end of the balloon. Despite good resistance to unwanted sudden movement, expansion performance at a diseased site of such balloons is subpar. If an artificial valve is not sufficiently expanded during use, paravalvular leakage and other complications tend to occur during its use.
Type II: A balloon of this type has a waist section, which will be fully expanded when the balloon is completely expanded. That is, during inflation of the balloon, it always comprises a figure-eight shape. However, when it is completely inflated, it will have a straight cylindrical shape instead, avoiding the problems that may arise from insufficient expansion. However, existing balloons of this type that can transform from a figure-eight shape to a straight cylindrical shape allows such shape transformation to occur only once. The conventional BAV or TAVI procedures typically require a balloon to be repeatedly inflated to ensure desirable surgical quality. If such a balloon can never comprise a figure-eight shape once it has been fully expanded, unwanted sudden movement will still tend to occur in the rest of the procedure.

### SUMMARY OF THE INVENTION

It is an object of the present invention to overcome the problem associated with conventional figure-eight shaped balloon expansion catheters that they are incapable of multiple reversible shape transformations, through presenting a novel expansion balloon and balloon expansion catheter.

To this end, the present invention provides an expansion e balloon comprising an outer balloon and an inner balloon,
the outer balloon sleeved over the inner balloon, the inner balloon switchable between a collapsed configuration, a pre-shaped configuration adapted to a predetermined expansion site and a first expanded configuration, the outer balloon having a second expanded configuration, wherein:
in case of an inflation fluid being filled into the inner balloon in a configuration between the collapsed configuration and the pre-shaped configuration, the inner balloon is expanded and transits from the collapsed configuration toward the pre-shaped configuration, with the outer balloon being expanded with an expansion the inner balloon; and in case of the inflation fluid being additionally filled into the inner balloon in the pre-shaped configuration, the inner balloon is further expanded, with the outer balloon being further expanded with an expansion of the inner balloon, until the outer balloon is expanded to the second expanded configuration, where the inner balloon is in the first expanded configuration.

Optionally, in case of the inflation fluid being evacuated from the inner balloon, the inner balloon may collapse and transit from the first expanded configuration toward the collapsed configuration, with the outer balloon collapsing with a collapse of the inner balloon.

Optionally, the outer balloon may be a non-compliant balloon, and the inner balloon may be a compliant balloon.

Optionally, in the expansion balloon, the inner balloon may sequentially comprise a first shoulder section, a waist section and a second shoulder section along an axis, wherein in case of the inner balloon being in the pre-shaped configuration, a radial dimension of the first shoulder section is greater than a radial dimension of the waist section, and a radial dimension of the second shoulder section is greater than the radial dimension of the waist section.

Optionally, in the expansion balloon, the inner balloon may further comprise two transition sections, one of the two transition sections is joined to each of the first shoulder section and the waist section, and the other of two transition sections is joined to each of the waist section and the second shoulder section.

Optionally, in the expansion balloon, the outer balloon may comprise a straight cylindrical section, wherein a ratio of an aggregate axial length of the first shoulder section, the waist section, the two transition sections and the second shoulder section to an axial length of the straight cylindrical section lies between 0.95 and 1.2.

Optionally, in the expansion balloon, a ratio of an axial length of the waist section to an aggregate axial length of the first shoulder section, the waist section and the second shoulder section may lie between 0 and 0.7.

Optionally, in the expansion balloon, in the pre-shaped configuration of the inner balloon, a ratio of an outer diameter of the waist section to a nominal diameter of the outer balloon may lie between 0.3 and 0.75. Additionally or alternatively, a ratio of an outer diameter of the first shoulder section or the second shoulder section to the nominal diameter of the outer balloon may lie between 0.6 and 1.0.

Optionally, in the expansion balloon, the outer balloon may comprise a straight cylindrical section, wherein in case of the outer balloon being in the second expanded configuration, the straight cylindrical section has a constant radial dimension.

Optionally, in the expansion balloon, a space between the outer balloon and the inner balloon may be pre-vacuumed so that the outer balloon and the inner balloon fit against each other.

Optionally, in the expansion balloon, a ratio of an axial length of the inner balloon to an axial length of the outer balloon in the collapsed configuration of the inner balloon may lie between 0.9 and 1.0.

To the above end, the present invention provides another expansion balloon comprising an outer balloon and an inner balloon, the outer balloon sleevedover the inner balloon, the outer balloon having a modulus of elasticity greater than 400 Mpa, the inner balloon having a modulus of elasticity smaller than 200 Mpa, wherein in case of the inflation fluid being filled into the inner balloon, the outer balloon is expanded, and wherein the inner balloon comprises a pre-shaped configuration during its expansion.

Optionally, in the balloon, the outer balloon may be made of at least one selected from PE, PET, PA and Pebax, and the inner balloon may be made of at least one selected from TPEE, TPU and silicone.

To the above end, the present invention provides a further expansion balloon comprising an outer balloon and an inner balloon, the outer balloon sleeved over the inner balloon, wherein in case of an inflation fluid being filled into the inner balloon, the outer balloon is expanded, and wherein the inner balloon comprises a pre-shaped configuration during its expansion, in which the inner balloon comprises a dumbbell-like shape that is narrower in the middle portion and wider at the end portions.

To the above end, the present invention provides an expansion balloon comprising an outer balloon and an inner balloon, the outer balloon sleeved over the inner balloon, wherein in case of an inflation fluid being filled into the inner balloon, the outer balloon is expanded, and wherein the inner balloon comprises a pre-shaped configuration during its expansion, in which an inflation pressure in the inner balloon lies between 0.1 atm and 2 atm.

To the above end, the present invention also provides balloon expansion catheter comprising the expansion balloon as defined above and a catheter, the catheter in communication with a proximal end of the inner balloon, the catheter used to transmit an inflation fluid to cause expansion or collapse of the expansion balloon.

In summary, the present invention provides an expansion balloon and a balloon expansion catheter. The expansion balloon includes an outer balloon and an inner balloon. The outer balloon is sleeved over the inner balloon, and the inner balloon can switch between a collapsed configuration, a pre-shaped configuration adapted to a predetermined expansion site and a first expanded configuration. The outer balloon has a second expanded configuration. In any intermediate configuration between the collapsed configuration and the pre-shaped configuration, when an inflation fluid is filled into the inner balloon, the inner balloon will transit from the collapsed configuration to the pre-shaped configuration, with the outer balloon being expanded with the inner balloon. In the pre-shaped configuration of the inner balloon, when the inflation fluid is additionally filled, the inner balloon will be further expanded, resulting in further expansion of the outer balloon, until the outer balloon is expanded to the second expanded configuration, where the inner balloon is in the first expanded configuration.

With this design, when in the pre-shaped configuration, the inner balloon can satisfy anchoring, partial expansion and special balloon shape requirements of various diseased sites. With the outer balloon defining a final expanded configuration of the expansion balloon, complications that may arise from excessive expansion can be avoided. Through combined use of the two balloons with different characteristics, the inner balloon can restore the original shape after repeated expansion of the expansion balloon and experience a similar configuration transitioning process in each expansion cycle (i.e., experiencing the pre-shaped configuration), and the final expanded configuration can be defined by the outer balloon (in the second expanded configuration), significantly reducing complications. Further, the inner and outer balloons have their own unique mechanical properties: the outer balloon can provide high puncture resistance and pressure resistance, and can limit the final expanded configuration of the expansion balloon; and the inner balloon can provide controllability over a shape transition process adapted to the predetermined expansion site.

### BRIEF DESCRIPTION OF THE DRAWINGS

Those of ordinary skill in the art would appreciate that the accompanying drawings are provided to facilitate a better understanding of the present invention and do not limit the scope thereof in any sense, in which:
Fig. 1 is a schematic illustration of a balloon expansion catheter according to an embodiment of the present invention;
Fig. 2 is a schematic illustration of an expansion balloon according to an embodiment of the present invention, in which an inner balloon is in a pre-shaped configuration;
Fig. 3 is a schematic axial cross-sectional view of an expansion balloon according to an embodiment of the present invention, in which an inner balloon is in a pre-shaped configuration;
Fig. 4 is a schematic illustration of an expansion balloon according to an embodiment of the present invention, in which an outer balloon is in a first expanded configuration;
Fig. 5 is a schematic transverse cross-sectional view of the expansion balloon of Fig. 4;
Fig. 6 is a schematic axial cross-sectional view of the expansion balloon of Fig. 4;
Fig. 7 is a schematic illustration of an expansion balloon according to an embodiment of the present invention, in which an inner balloon is in a collapsed configuration; and
Fig. 8 is a schematic transverse cross-sectional view of the expansion balloon of Fig. 7.

In these figures,
1, an expansion balloon; 4, a guidewire lumen; 5, a connecting member; 6, a radiopaque ring; 7, a catheter; 71, an inner tube; 72, an outer tube; 8, a protective sheath; 9, a fluid lumen;
10, an outer balloon; 11, a first conical section; 12, a straight cylindrical section; 13, a second conical section;
20, an inner balloon; 21, a third conical section; 22, a first shoulder section; 23, a first transition section; 24, a waist section; 25, a second transition section; 26, a second shoulder section; and 27, a fourth conical section.

### DETAILED DESCRIPTION

Objects, advantages and features of the present invention will become more apparent upon reading the following more detailed description of the present invention, which is made with reference to the accompanying drawings. Note that the figures are provided in a very simplified form not necessarily drawn to exact scale and for the only purpose of facilitating easy and clear description of the embodiments. In addition, structures shown in the figures are usually a part of actual structures. In particular, as the figures tend to have distinct emphases, they are often drawn to different scales.

As used herein, the singular forms "a", "an" and "the" include plural referents, unless the context clearly dictates otherwise. As used herein, the term "or" is generally employed in the sense of "and/or", "several" is generally employed in the sense of "at least one" and "at least two" is generally employed in the sense of "two or more", unless the context clearly dictates otherwise. Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal end" generally refers to an end closer to an operator, and the term "distal end" generally refers to an end closer to a lesion in a patient. The terms "one end" and "the other end", as well as "proximal end" and "distal end", are generally used to refer to opposite end portions including the opposite endpoints, rather than only to the endpoints. The terms "mounting", "coupling" and "connection" should be interpreted in a broad sense. For example, a connection may be a permanent, detachable or integral connection, or a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location with respect to the other element, unless the context clearly dictates otherwise. Those of ordinary skill in the art can understand the specific meanings of the above-mentioned terms herein, depending on their context.

In principle, the present invention seeks to overcome the problem associated with conventional figure-eight shaped balloon expansion catheters that they are incapable of multiple reversible shape transformations, through presenting a novel expansion balloon and balloon expansion catheter.

This invention will be described below with reference to the accompanying drawings.

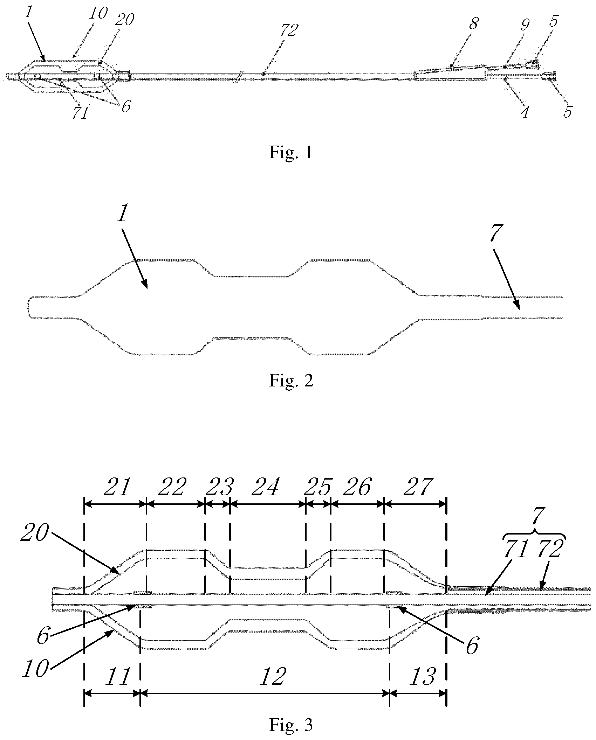

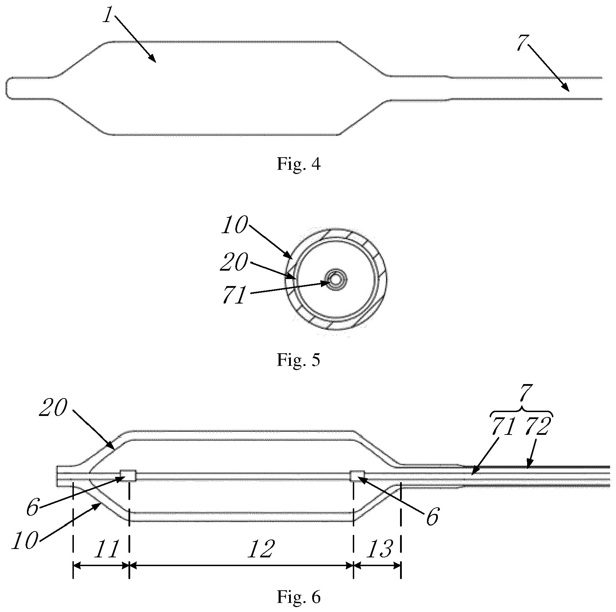

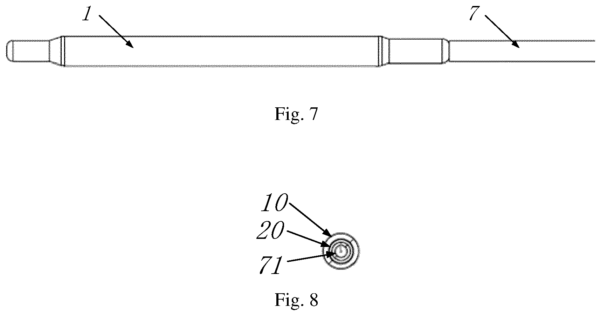

Reference is now made to Figs. 1 to 8. Fig. 1 is a schematic illustration of a balloon expansion catheter according to an embodiment of the present invention. Fig. 2 is a schematic illustration of an expansion balloon according to an embodiment of the present invention, in which an inner balloon is in a pre-shaped configuration. Fig. 3 is a schematic axial cross-sectional view of an expansion balloon according to an embodiment of the present invention, in which an inner balloon is in a pre-shaped configuration. Fig. 4 is a schematic illustration of an expansion balloon according to an embodiment of the present invention, in which an outer balloon is in a first expanded configuration. Fig. 5 is a schematic transverse cross-sectional view of the expansion balloon of Fig. 4. Fig. 6 is a schematic axial cross-sectional view of the expansion balloon of Fig. 4. Fig. 7 is a schematic illustration of an expansion balloon according to an embodiment of the present invention, in which an inner balloon is in a collapsed configuration. Fig. 8 is a schematic transverse cross-sectional view of the expansion balloon of Fig. 7.

As shown in Figs. 1 and 2, in a first embodiment of the present invention, there is provided a balloon expansion catheter including an expansion balloon 1 and a catheter 7. The expansion balloon 1 includes an outer balloon 10 and an inner balloon 20. The catheter 7 communicates with a proximal end of the inner balloon 20 and is used to transmit an inflation fluid to cause expansion or collapse of the expansion balloon 1. The outer balloon 10 is sleeved over the inner balloon 20, and the inner balloon 20 is able to switch between a collapsed configuration, a pre-shaped configuration adapted to a predetermined expansion site and a first expanded configuration. The outer balloon 10 has a second expanded configuration. As a result of the inflation fluid being filled, the inner balloon 20 will transition from the collapsed configuration to the pre-shaped configuration, and the outer balloon 10 will be expanded with the expansion of the inner balloon 20. In the pre-shaped configuration of the inner balloon 20, when the inflation fluid is additionally filled, the inner balloon 20 will be further expanded, resulting in further expansion of the outer balloon 10, until the outer balloon 10 is expanded to the second expanded configuration, where the inner balloon 20 is in the first expanded configuration. It is to be noted that, here, by "the outer balloon 10 is sleeved over the inner balloon 20", it is intended to mean that, in a certain radial cross-section of the expansion balloon 1, the distance from the outer balloon 10 to an axis of the expansion balloon 1 is greater than the distance from the inner balloon 20 to the axis of the expansion balloon 1. On the contrary, it would be appreciated that, when a component is referred to as being "disposed within" another component, it is intended to mean that the distance from the inner component to the axis is smaller than the distance from the outer component to the axis.

In addition, in the first expanded configuration of the inner balloon 20, as a result of evacuating the inflation fluid therefrom, the inner balloon 20 will collapse toward the collapsed configuration, causing simultaneous collapse of the outer balloon 10 therewith. The collapse of the inner balloon 20 resulting from the evacuation of the inflation fluid is a reverse process of the expansion resulting from the filling of the inflation fluid. In particular, fast evacuation may cause the inner balloon 20 to rapidly transit from the first expanded configuration to the collapsed configuration without necessarily undergoing the pre-shaped configuration. The expansion balloon 1 allows the inner balloon 20 to be repeatedly inflated and deflated, causing repeated expansion and collapse of the expansion balloon 1.

Preferably, the outer balloon 10 is a non-compliant balloon, and the inner balloon 20 is a compliant balloon. It is to be noted that, for any single balloon (e.g., the outer balloon 10 or the inner balloon 20), when the inflation fluid (e.g., an inflation liquid) is filled into the balloon to a nominal pressure (i.e., a rated pressure), the balloon will expand to a predetermined size. In general, at this time, the balloon has a substantially round cross-section and a (outer) diameter is its nominal diameter. At the nominal diameter, if the balloon is further filled with the inflation fluid, it will continue expanding and eventually burst at a certain time point. The internal pressure at the time when the balloon bursts is defined as its rated burst pressure. Herein, a compliant balloon is defined as a balloon with a diameter at its rated burst pressure, which is 30% greater than its nominal diameter, and a non-compliant balloon is defined as a balloon with a diameter at the rated burst pressure, which is not 15% greater than its nominal diameter.

Because the outer balloon 10 may come into direct contact with human tissues during use, it is desired to have sufficient puncture and pressure resistance, which can prevent it from being punctured by some human tissues such as a calcified valvular annulus. For this reason, the outer balloon 10 is preferably chosen as a non-compliant balloon. As the inner balloon 20 is disposed within the outer balloon 10 and desired to have good elastic deformability, it is preferably chosen as a compliant balloon. The inner balloon 20 can expand noticeably as an inflation pressure is increasing. Moreover, the inner balloon 20 may be pre-shaped according to the anatomical geometry at a predetermined expansion site (i.e., a diseased site). For example, when the expansion balloon 1 is intended for use for the treatment of heart valve disease, for example, in an aortic valve produce, a predetermined configuration of the inner balloon 20 may comprise a dumbbell-like shape, i.e., narrower in the middle and wider at the ends. This enables, inter alia, attachment (or anchoring) of the inner balloon 20 at the diseased site, and partial expansion of the diseased site. Compared with the inner balloon 20, the outer balloon 10 shows significantly higher pressure resistance, and its material has significantly stronger mechanical properties (modulus of elasticity or strength).

The inventors have found that, when encountering resistance within a blood vessel, a compliant balloon tends to deform so as to be more expanded where it is less resisted. One consequence of this is a significant reduction in squeezing strength on the surrounding diseased tissue. Another consequence is that a continuous inflation pressure rise will lead to excessive expansion of shoulder portions of the balloon at opposite sides thereof, which may cause damage or even dissection of the wall of the blood vessel. Since compliant balloons tend to cause dissection when used to expand a stenotic lesion, they are generally considered not suitable for use in angioplasty. Moreover, for valvuloplasty, excessive expansion of a balloon may possibly cause tearing of a native annulus, which may in turn cause serious complications. Therefore, compliant balloons are also generally considered not suitable for use in valvuloplasty. In contrast, through combined use of the outer balloon 10 and the inner balloon 20, the non-compliant outer balloon 10 can define a final expanded configuration of the expansion balloon 1, circumventing the problems of easy excessive expansion and possible occurrence of complications that may arise from the use of a single compliant balloon. Meanwhile, the compliant inner balloon 20 enables the expansion balloon 1 to restore the original shape after repeated expansion.

An exemplary balloon expansion catheter constructed in accordance with the present embodiment will be described with reference to Figs. 1, 3 and 5. It would be appreciated that Fig. 1 is not intended to depict actual use of the balloon expansion catheter but to schematically illustrate its structural composition.

Optionally, the catheter 7 may be a double-layered structure including an inner tube 71 and an outer tube 72. The outer tube 72 is sleeved over the inner tube 71, and a space is provided between the inner tube 71 and the outer tube 72 for passage of an inflation fluid therethrough. The inner tube 71 protrudes out of the outer tube 72 from a distal end thereof into the inner balloon 20, and a distal end of the inner balloon 20 is fixedly and sealingly connected to the inner tube 71. A distal end of the outer balloon 10 is fixedly and sealingly connected to the distal end of the inner balloon 20. A proximal end of the inner balloon 20 is fixedly and sealingly connected to the outer tube 72, and a proximal end of the outer balloon 10 is fixedly and sealingly connected to the proximal end of the inner balloon 20. The inner tube 71 is configured essentially for insertion of a guidewire therethrough. Optionally, the inner tube 71 may be provided thereon with a radiopaque ring 6. When the inflation fluid is conveyed through the catheter 7 (more precisely, through the space between the inner tube 71 and the outer tube 72) to the inner balloon 20, the inner balloon 20 and the outer balloon 10 will be expanded. On the contrary, when the inflation fluid is evacuated from the inner balloon 20 through the catheter 7, the inner balloon 20 and the outer balloon 10 will collapse. Preferably, a space between the outer balloon 10 and the inner balloon 20 is pre-vacuumed so that the outer balloon 10 closely fits against the inner balloon 20 and they expand and collapse in synchronization during expansion and collapse of the expansion balloon 1. This can facilitate configuration control of the expansion balloon 1. It is to be noted that, here, the term "pre-vacuumed" is intended to be interpreted in a broad sense as "vacuumed to a negative pressure compared to the atmospheric pressure", rather than in a narrow sense as "absolutely vacuumed". Limited by current processes, the term "vacuum" is intended to refer to a rough vacuum (10-760 Torr) or a moderate vacuum (1-10 Torr).

The balloon expansion catheter may further comprise accessories such as a protective sheath 8, a fluid lumen 9, a guidewire lumen 4 and connecting members 5. The protective sheath 8 may be provided with a tee, to which the fluid lumen 9, the guidewire lumen 4 and the catheter 7 are all coupled. Optionally, the guidewire lumen 4 and the inner tube 71 of the catheter 7 may be both connected to a trunk of the tee, and they communicate with each other and comprise an in-line configuration. The fluid lumen 9 may be connected to a branch of the tee and communicates with the outer tube 72 of the catheter 7. Each of the fluid lumen 9 and the guidewire lumen 4 may be connected at a proximal end to one of the connecting members and hence to an associated external component. Those skilled in the art may properly choose and configure the various components in the balloon expansion catheter according to conventional knowledge in the art.

In a preferred embodiment, the outer balloon 10 includes a first conical section 11, a straight cylindrical section 12 and a second conical section 13, which are sequentially joined together along an axis of the outer balloon 10. In the expanded configuration of the outer balloon 10, the first conical section 11 and the second conical section 13 both comprise substantially conical shapes, and the straight cylindrical section 12 comprises a substantially straight cylindrical shape. The outer balloon 10 may have the same shape as any mainstream balloon. Those skilled in the art would understand its configuration based on conventional knowledge in the art. Referring to Figs. 4 to 6, when the outer balloon 10 is in the second expanded configuration, the straight cylindrical section 12 may have a constant radial dimension. It would be appreciated that, in the course of expansion of the expansion balloon 1, the straight cylindrical section 12 may not necessarily keep a constant radial dimension at any time. During expansion of the inner balloon 20 from the collapsed configuration to the pre-shaped configuration, the outer balloon 10 and the inner balloon 20 may be kept substantially similar in shape, and the straight cylindrical section 12 may have a similar shape to that of the inner balloon 20, such as a dumbbell-like shape.

Correspondingly, the inner balloon 20 may include a third conical section 21 and a fourth conical section 27 at its opposite ends, which match the first conical section 11 and the second conical section 13 in shape. When the inner balloon 20 is expanded (e.g., in the pre-shaped configuration or the second expanded configuration), both the third conical section 21 and the fourth conical section 27 comprise substantially conical shapes matching the respective shapes of the first conical section 11 and the second conical section 13.

Further, referring to Figs. 2 and 3, the expansion balloon 1 may be used for treatment of heart valve disease, for example. The inner balloon 20 includes a first shoulder section 22, a waist section 24 and a second shoulder section 26, which are sequentially arranged axially. In the pre-shaped configuration of the inner balloon 20, the first shoulder section 22 has a radial dimension greater than a radial dimension of the waist section 24, and the second shoulder section 26 also has a radial dimension greater than the radial dimension of the waist section 24. A distal end of the first shoulder section 22 is joined to a proximal end of the third conical section 21, and a proximal end of the second shoulder section 26 is joined to a distal end of the fourth conical section 27. The radial dimensions here refer to distances from one side to the opposite side of the outer side wall of the inner balloon 20 and pass through an axis of the inner balloon 20. Optionally, the inner balloon 20 may be cross-sectionally circular. Accordingly, the radial dimensions of the various sections of the inner balloon 20 refer to their diameters. The first shoulder section 22, the waist section 24 and the second shoulder section 26 are all straight cylindrical. Preferably, the diameter of the first shoulder section 22 is equal to that of the second shoulder section 26. Due to a predefined geometrical design, when the inner balloon 20 is partially inflated, it comprises a dumbbell-like shape as shown in Fig. 2. The waist section 24 can be anchored at a valvular annulus, preventing the expansion balloon 1 shifting away from the lesion due to unwanted sudden movement.

Referring to Figs. 7 and 8, in the collapsed configuration of the inner balloon 20, the outer balloon 10 is collapsed and folded accordingly. As a result, the entire expansion balloon 1 has a relatively small outer diameter that facilitates passage of the expansion ion balloon 1 through a blood vessel. Preferably, the inner balloon 20 has a small wall thickness. For example, the wall thickness of the inner balloon 20 may range from 0.005 mm to 0.1 mm and is more preferred to be 0.01 mm. The small wall thickness of the inner balloon 20 allows the expansion balloon 1 to have a small overall outer diameter. In the collapsed configuration of the inner balloon 20, when the inflation fluid is filled therein, the inner balloon 20 will gradually transition toward the pre-shaped configuration, with the outer balloon 10 surrounding the inner balloon 20 being concurrently expanded. On the contrary, in the pre-shaped configuration of the inner balloon 20, when the inflation fluid is evacuated therefrom, the inner balloon 20 will gradually collapse toward the collapsed configuration, with the outer balloon 10 collapsing concurrently.

Further, in the pre-shaped configuration of the inner balloon 20, when the inflation fluid is additionally filled therein, the inner balloon 20 will further expand. However, limited by the outer balloon 10, the waist section 24 of the inner balloon 20 will be expanded faster than the first shoulder section 22 and the second shoulder section 26, until an outer diameter of the waist section 24 becomes equal to that of the first shoulder section 22 and the second shoulder section 26. When this happens, limited by the straight cylindrical section 12 of the outer balloon 10, the three sections will be uniformly expanded instead. That is, at this time, the first shoulder section 22, the waist section 24 and the second shoulder section 26 generally appear as a straight cylinder. In some embodiments, upon the outer diameter of the waist section 24 becoming equal to that of the first shoulder section 22 and the second shoulder section 26, the inner balloon 20 is considered as being in the first expanded configuration, the outer balloon 10 as being in the second expanded configuration, and the entire expansion balloon 1 as comprising its nominal diameter. In some other embodiments, after the outer diameter of the waist section 24 becomes equal to that of the first shoulder section 22 and the second shoulder section 26, the inflation fluid may be additionally filled into the inner balloon 20 to cause overall expansion of the expansion balloon 1 until the expansion balloon 1 reaches its nominal diameter. At this time, the inner balloon 20 is in the first expanded configuration, and the outer balloon 10 is in the second expanded configuration. It would be appreciated that collapse of the expansion balloon 1 resulting from evacuating the inflation fluid from the inner balloon 20 is a reverse process of the expansion process as described above. Those skilled in the art can understand the collapse process in light of the above description, and further description thereof is deemed unnecessary. When the outer diameter of the waist section 24 is equal to that of the first shoulder section 22 and the second shoulder section 26, the entire expanded expansion balloon 1 will resemble a conventional straight cylindrical balloon. This design is more favorable for a surgeon to choose, based on the size(s) of diseased native leaflets and/or an annulus, a balloon size suitable for faster and more uniform pre-expansion in a BAV or TAVI procedure. Thus, higher TAVI quality can be obtained by sufficiently dilating an implanted bioprosthetic aortic valve, without causing paravalvular leakage or other complications.

Optionally, the inner balloon 20 may further include two transition sections: a first transition section 23 and a second transition section 25. One of the transition sections (i.e., the first transition section 23) is joined to both the first shoulder section 22 and the waist section 24, and the other transition section (i.e., the second transition section 25) is joined to both the waist section 24 and the second shoulder section 26. This arrangement enables smooth transitioning between the shoulder sections and the waist section 24. In case of different radial dimensions of the shoulder sections and the waist section 24, each transition section is preferably in the shape of a cone with a diametrically smaller end joined to the waist section 24 and a diametrically larger end joined to the shoulder section.

Optionally, a ratio of an aggregate axial length of the first shoulder section 22, the waist section 24, the two transition sections and the second shoulder section 26 to an axial length of the straight cylindrical section 12 is in the range of 0.95 to 1.2, preferably 1.0. Considering the compliance difference between the inner balloon 20 and the outer balloon 10, a ratio selected from the above range allows the inner and outer balloons to better fit against each other during the expansion and collapse processes without warpage, peeling or other problems. Optionally, the aforementioned aggregate axial length of the first shoulder section 22, the waist section 24, the two transition sections and the second shoulder section 26 may be an axial length of the inner balloon 20 in the collapsed configuration. It is to be noted that the length of the inner balloon 20 in the collapsed configuration refers to an initial length thereof before it is assembled with the outer balloon 10. Specifically, during the assembly, the inner balloon 20 in the collapsed configuration may be stretched so that its proximal and distal ends are aligned with the proximal and distal ends of the outer balloon 10 in the collapsed configuration, respectively. Thus, after being assembled, the inner balloon 20 remains in an axially stretched state. Further, in the collapsed configuration of the inner balloon 20, an axial length ratio of the inner balloon 20 to the outer balloon 10 may be in the range of 0.9-1.0, preferably 0.98. That is, the axial length of the outer balloon 10 is approximately similar to that of the inner balloon 20. In general, the length of the outer balloon 10 is slightly larger than the initial length of the inner balloon 20 (i.e., the axial length of the inner balloon 20 in the collapsed configuration). Given different radial and axial expansion abilities of the outer balloon 10 and inner balloon 20 when they are inflated due to their different compliance levels, this design can ensure more compatible lengths of them during the inflation (including the conditions where the inner balloon 20 is in the pre-shaped configuration, the first expanded configuration and any intermediate configuration therebetween). Optionally, an initial outer diameter of the straight cylindrical section 12 of the outer balloon 10 (i.e., an outer diameter of the outer balloon 10 when the inner balloon 20 is in the collapsed configuration) may be greater than or equal to an initial outer diameter of the shoulder sections of the inner balloon 20.

Preferably, a ratio of an axial length of the waist section 24 to an aggregate axial length of the first shoulder section 22, the waist section 24 and the second shoulder section 26 ranges from 0 to 0.7. More preferably, a ratio of the axial length of the waist section 24 to the aggregate axial length of the first shoulder section 22, the waist section 24, the two transition sections and the second shoulder section 26 ranges from 0 to 0.7. When either of the ratios is close to 0, the inner balloon 20 (in the pre-shaped configuration) will comprise a figure-eight shape rather than a dumbbell-like one. Preferably, the ratio of the axial length of the waist section 24 to the aggregate axial length of the first shoulder section 22, the waist section 24, the two transition sections and the second shoulder section 26 is 0.35. Optionally, in the pre-shaped configuration of the inner balloon 20, a ratio of the outer diameter of the waist section 24 to the nominal diameter of the outer balloon 10 may lie between 0.3 and 0.75 and is preferred to be 0.55. Additionally or alternatively, a ratio of the outer diameter of the first shoulder section 22 and the second shoulder section 26 to the nominal diameter of the outer balloon 10 may lie between 0.6 and 1.0 and is preferred to be 0.8.

Furthermore, the inflation pressure for the inner balloon 20 in the pre-shaped configuration may be chosen and configured as needed. The inventors have found that, in the pre-shaped configuration, the inflation pressure for the inner balloon 20 depends on an initial diameter difference between the inner and outer balloons, a diameter difference between the waist section 24 and the shoulder sections in the pre-shaped configuration of the inner balloon 20 and the moduli of elasticity of the inner and outer balloons. Preferably, taking into suitable values of the above parameters, the inflation pressure for the inner balloon 20 may be selected from the range of 0.1 atm to 2 atm.

A particular embodiment of the expansion balloon 1 will be described below. It would be appreciated that the data disclosed hereunder are merely those of a preferred example of the expansion balloon 1 and are not intended to limit the expansion balloon 1 in any sense.

The axial length of the straight cylindrical section 12 in the outer balloon 10 (at the nominal pressure of the outer balloon 10) is in the range of 20-55 mm, preferably 36 mm. Axial lengths of the first conical section 11 and the second conical section 13 vary with the diameter of the outer balloon 10, generally in the range of 8-60 mm. Angles at conical tips of the first conical section 11 and the second conical section 13 (i.e., angles formed by conical surfaces of the first conical section 11 and the second conical section 13 with respect to the axis of the outer balloon 10) are in the range of 25° to 100°, preferably 45°.

The size of the outer balloon 10 is selected to correspond to a standard balloon size (balloons are generally standardized according to their materials, dimensions and expansion performance into various standard sizes, and conventional common expansion balloons usually have a size of 8 mm to 34 mm) lies between 14 mm and 26 mm. The inventors have found that the outer balloon 10 with a such size can cooperate with the inner balloon 20 to exhibit good resistance to unwanted sudden movement.

The modulus of elasticity of the outer balloon 10 may be greater than 400 Mpa, preferably greater than 1500 Mpa. A material for the outer balloon 10 may be selected as one or a combination of macromolecular polymers such as PE, PET, PA and Pebax. Such a material enables it to have good non-compliance, high puncture resistance and a certain degree of pressure resistance. The present invention is not limited to any particular material, color, layered or braided structure of the outer balloon 10. Those skilled in the art may select a suitable material for the outer balloon 10 according to conventional knowledge in the art.

A material for the inner balloon 20 may be selected as at least one of a thermoplastic elastomer such as TPEE or TPU and silicone (essentially elastomeric silicone with a breaking elongation rate greater than 400%). The modulus of elasticity of the inner balloon 20 may be smaller than 200 Mpa, preferably smaller than 50 Mpa.

In summary, the present invention provides an expansion balloon and a balloon expansion catheter. The expansion balloon includes an outer balloon and an inner balloon. The outer balloon is sleeved over the inner balloon, and the inner balloon can switch between a collapsed configuration, a pre-shaped configuration adapted to a predetermined expansion site and a first expanded configuration. The outer balloon has a second expanded configuration. In any intermediate configuration between the collapsed configuration and the pre-shaped configuration, when an inflation fluid is filled into the inner balloon, the inner balloon will transition from the collapsed configuration to the pre-shaped configuration, with the outer balloon being expanded with the inner balloon. In the pre-shaped configuration of the inner balloon, when the inflation fluid is additionally filled, the inner balloon will be further expanded, resulting in further expansion of the outer balloon, until the outer balloon is expanded to the second expanded configuration, where the inner balloon is in the first expanded configuration.

With this design, when in the pre-shaped configuration, the inner balloon can satisfy anchoring of various diseased sites, partial expansion of various diseased sites and special balloon shape requirements. With the outer balloon defining a final expanded configuration of the expansion balloon, complications that may arise from excessive expansion can be avoided. Through combined use of the two balloons with different characteristics, the inner balloon can restore the original shape after repeated expansion of the expansion balloon and experience a similar configuration transitioning process in each expansion cycle (i.e., experiencing the pre-shaped configuration), and the final expanded configuration can be defined by the outer balloon (in the second expanded configuration), significantly reducing complications. Further, the inner and outer balloons have their own unique mechanical properties: the outer balloon can provide high puncture resistance and pressure resistance, and can limit the final expanded configuration of the expansion balloon; and the inner balloon can provide controllability over a shape transition process adapted to the predetermined expansion site.

It is to be noted that the embodiments disclosed herein are described in a progressive manner, with the description of each embodiment focusing on its differences from others. Reference can be made between the embodiments for their identical or similar features. Additionally, features of different embodiments may be combined with one another, without limiting the scope of the present invention. The description presented above is merely that of some preferred embodiments of the present invention and does not limit the scope thereof in any sense. Any and all changes and modifications made by those of ordinary skill in the art based on the above teachings fall within the scope as defined in the appended claims.

## Claims

1. An expansion balloon, comprising an outer balloon and an inner balloon, wherein the outer balloon is sleeved over the inner balloon, wherein the inner balloon is switchable between a collapsed configuration, a pre-shaped configuration and a first expanded configuration, wherein the outer balloon has a second expanded configuration, and wherein:
in case of an inflation fluid being filled into the inner balloon in a configuration between the collapsed configuration and the pre-shaped configuration, the inner balloon is expanded and transits from the collapsed configuration toward the pre-shaped configuration, with the outer balloon being expanded with an expansion the inner balloon; and in case of the inflation fluid being additionally filled into the inner balloon in the pre-shaped configuration, the inner balloon is further expanded, with the outer balloon being further expanded with an expansion of the inner balloon, until the outer balloon is expanded to the second expanded configuration, where the inner balloon is in the first expanded configuration.

2. The expansion balloon according to claim 1, wherein in case of the inflation fluid being evacuated from the inner balloon, the inner balloon collapses and transits from the first expanded configuration toward the collapsed configuration, with the outer balloon collapsing with a collapse of the inner balloon.

3. The expansion balloon according to claim 1, wherein the outer balloon is a non-compliant balloon, and wherein the inner balloon is a compliant balloon.

4. The expansion balloon according to claim 1, wherein the inner balloon sequentially comprises a first shoulder section, a waist section and a second shoulder section along an axis, wherein in case of the inner balloon being in the pre-shaped configuration, a radial dimension of the first shoulder section is greater than a radial dimension of the waist section, and a radial dimension of the second shoulder section is greater than the radial dimension of the waist section.

5. The expansion balloon according to claim 4, wherein the inner balloon further comprises two transition sections, wherein one of the two transition sections is joined to each of the first shoulder section and the waist section, and wherein the other one of the two transition sections is joined to each of the waist section and the second shoulder section.

6. The expansion balloon according to claim 5, wherein the outer balloon comprises a straight cylindrical section, wherein a ratio of an aggregate axial length of the first shoulder section, the waist section, the two transition sections and the second shoulder section to an axial length of the straight cylindrical section lies between 0.95 and 1.2.

7. The expansion balloon according to claim 5, wherein a ratio of an axial length of the waist section to an aggregate axial length of the first shoulder section, the waist section and the second shoulder section lies between 0 and 0.7.

8. The expansion balloon according to claim 5, wherein in case of the inner balloon being in the pre-shaped configuration, a ratio of an outer diameter of the waist section to a nominal diameter of the outer balloon lies between 0.3 and 0.75, and/or wherein a ratio of an outer diameter of the first shoulder section or the second shoulder section to the nominal diameter of the outer balloon lies between 0.6 and 1.0.

9. The expansion balloon according to claim 1, wherein the outer balloon comprises a straight cylindrical section, wherein in case of the outer balloon being in the second expanded configuration, the straight cylindrical section has a constant radial dimension.

10. The expansion balloon according to claim 1, wherein a space between the outer balloon and the inner balloon is pre-vacuumed so that the outer balloon and the inner balloon fit against each other.

11. The expansion balloon according to claim 1, wherein in case of the inner balloon being in the collapsed configuration, a ratio of an axial length of the inner balloon to an axial length of the outer balloon lies between 0.9 and 1.0.

12. An expansion balloon, comprising an outer balloon and an inner balloon, wherein the outer balloon is sleeved over the inner balloon, wherein the outer balloon has a modulus of elasticity greater than 400 Mpa, wherein the inner balloon has a modulus of elasticity smaller than 200 Mpa, wherein in case of the inflation fluid being filled into the inner balloon, the outer balloon is expanded, and wherein the inner balloon comprises a pre-shaped configuration during an expansion thereof.

13. The expansion balloon according to claim 12, wherein the outer balloon is made of at least one selected from PE, PET, PA and Pebax, and wherein the inner balloon is made of at least one selected from TPEE, TPU and silicone.

14. An expansion balloon, comprising an outer balloon and an inner balloon, wherein the outer balloon is sleeved over the inner balloon, wherein in case of an inflation fluid being filled into the inner balloon, the outer balloon is expanded, and wherein the inner balloon comprises a pre-shaped configuration during an expansion thereof, in which the inner balloon comprises a dumbbell-like shape that is narrower in a middle portion and wider at end portions.

15. An expansion balloon, comprising an outer balloon and an inner balloon, wherein the outer balloon is sleeved over the inner balloon, wherein in case of an inflation fluid being filled into the inner balloon, the outer balloon is expanded, and wherein the inner balloon comprises a pre-shaped configuration during an expansion thereof, in which an inflation pressure in the inner balloon lies between 0.1 atm and 2 atm.

16. An balloon expansion catheter, comprising the expansion balloon of any one of claims 1 to 15 and a catheter, wherein the catheter is in communication with a proximal end of the inner balloon, and wherein the catheter is used to transmit an inflation fluid to cause an expansion or a collapse of the expansion balloon.
